# EUROPÄISCHE PATENTSCHRIFT

(11) **EP 3 389 389 B1**
(45) Veröffentlichungstag und Bekanntmachung des Hinweises auf die Patenterteilung: **27.03.2024**
(21) Anmeldenummer: 16805103.5
(22) Anmeldetag: 01.12.2016
(51) Int. Cl.: A61P 3/02, A23G 3/42, A23G 3/44, A23L 29/281

(54) **GIESSMASSE UND VERFAHREN FÜR DIE HERSTELLUNG VON GELATINEPRODUKTEN**
CASTING COMPOUND AND PROCESS FOR THE PRODUCTION OF GELATIN PRODUCTS
MASSE DE COULÉE ET PROCÉDÉ POUR LA FABRICATION DE PRODUITS GÉLATINEUX

(30) Priorität: 16.12.2015 DE 102015121923
(43) Veröffentlichungstag der Anmeldung: 24.10.2018
(73) Patentinhaber: Gelita AG, 69412 Eberbach (DE)
(72) Erfinder: DICK, Eberhard, 69151 Neckargemünd (DE); GÖTTLING, Sonja, 64646 Heppenheim (DE); LEITHEIM, Anna, 74821 Mosbach (DE); RAAB, Alexander, 69239 Neckarsteinach (DE); BRACK, Holger, 55452 Rümmelsheim (DE)
(74) Vertreter: Hoeger, Stellrecht & Partner Patentanwälte mbB
(86) Internationale Anmeldenummer: PCT/EP2016/079388
(87) Internationale Veröffentlichungsnummer: WO 2017/102347

(56) Entgegenhaltungen:
- EP-A1- 2 724 623
- WO-A1-97/41738
- WO-A1-98/12935
- WO-A1-2012/006215
- WO-A2-2004/056976
- WO-A2-2009/072817
- DE-A1-102007 007 307
- DE-A1-102011 056 018
- US-A1- 2004 013 732
- US-A1- 2007 148 292
- US-A1- 2015 216 199

## Beschreibung

Die vorliegende Erfindung betrifft eine Gießmasse für die Herstellung von Gelatineprodukten.

Die Erfindung betrifft ferner ein Verfahren zur Herstellung von Gelatineprodukten unter Verwendung dieser Gießmasse, sowie die aus ihr hergestellten Gelatineprodukte.

Unter den Oberbegriff der Gelatineprodukte, wie er im Folgenden verwendet wird, fallen zum einen beliebte Zuckersüßwaren, die sich in erster Linie durch eine mehr oder weniger elastische Textur auszeichnen. Neben der Gelatine bilden verschiedene Zuckerarten und/oder Zuckeraustauschstoffe den Hauptbestandteil dieser Produkte, die im weitesten Sinne als Gummibonbons bezeichnet werden können, und insbesondere in Form von Gummibären oder Fruchtgummis bekannt sind.

US2004/013732 A1 offenbart Gelatineprodukte mit folgenden Bestandteilen: 5.51 Gew.% Gelatine, 15.75 Gw.% Glycerol. Die Gelatine ist eine hoch Molekulargewicht Gelatine. Die Wasseraktivität ist 0.45-0.55 (weniger als 0.7) und Wasser Enthalt ist 13-20%. Die Gießlösung ist benutzt für der Herstellung von Gelatineprodukten.

Zum anderen werden solche Gelatineprodukte auch als Kautabletten im Bereich der Nahrungsergänzungsmittel und der Arzneimittel eingesetzt, wobei der Grundrezeptur verschiedene Nährstoffe (z.B. Vitamine, Mineralstoffe oder Peptide) bzw. pharmazeutische Wirkstoffe zugesetzt sind. Der Zuckergehalt kann in diesem Fall reduziert sein, wobei der Übergang von Süßwaren zu Nahrungsergänzungsmitteln durchaus fließend ist. Gummibonbons bzw. Kautabletten, die mit verschiedenen Zusatz- oder Wirkstoffen angereichert sind, werden z.B. als sogenannte "Fortified Gummies" angeboten.

Das bisher eingesetzte Herstellungsverfahren für solche Gelatineprodukte ist als Mogul-Technik bekannt. Bei diesem Verfahren wird eine heiße Gießmasse mit einem Wassergehalt von ca. 25 Gew.%, welche die Gelatine, den Zucker und die übrigen Bestandteile in Wasser gelöst enthält, in Hohlformen aus einem Stärkeformpuder gegossen. Die Hohlformen werden zuvor durch Eindrücken einer positiv Form in die glatte Oberfläche eines mit trocknem Stärkepuder gefüllten, flachen Kastens erzeugt. Nach dem Befüllen der Hohlformen werden die Stärkepuderkästen zwischen 24 und 72 Stunden in einem Klimaraum gelagert. Während dieser Zeit kühlt die Gießmasse in den Hohlformen ab, was zu einem Gelieren der gegossenen Formkörper führt. Parallel dazu wird ein Teil des Wassers von dem Stärkeformpuder aufgenommen, so dass eine Trocknung stattfindet, wobei die fertigen Gelatineprodukte in der Regel einen Wassergehalt von etwa 20 Gew.% oder weniger aufweisen. Anschließend werden die Puderkästen entleert, das Stärkeformpuder mittels Sieben von den Gelatineprodukten abgetrennt und nach einer Trocknung wiederverwendet. Die Gelatineprodukte werden mit einem Trennmittel behandelt ("Beölen"), um ein Verkleben zu verhindern, und verpackt.

Die Verwendung von Stärkeformpuder für die Erzeugung der Hohlformen ist mit verschiedenen Nachteilen verbunden. Aufgrund der langen Trocknungszeit (24 bis 72 Stunden) der mit den bekannten Rezepturen in Stärkeformpuder gegossenen Gelatineprodukte werden bei entsprechend leistungsfähigen Mogul-Anlagen, die bis zu 35 Puderkästen pro Minute gießen, große Trocknungskammern und eine sehr hohe Anzahl an Formpuderkästen benötigt. Der Raumbedarf und die notwendigen Investitionen für Klimatisierung, Formpuderkästen, Stärketrockner und nicht zuletzt das Stärkeformpuder sind daher erheblich. Problematisch ist auch die Verunreinigung der Produktionsräume mit Stärkepuder, welche trotz permanenter Reinigung nicht komplett zu vermeiden ist.

Ein weiterer Nachteil des Mogul-Verfahrens ist die Gefahr einer Kreuzkontamination bei einem Produktwechsel, da stets Verunreinigungen des zuvor produzierten Produktes in der Stärke verbleiben, welche bei der kontinuierlichen Wiederverwendung des Stärkeformpuders in das neue Produkt eingeschleppt werden können. Dieses Problem könnte nur durch das vollständige Verwerfen des Stärkeformpuders vor jedem Produktwechsel vermieden werden, was jedoch völlig unwirtschaftlich wäre.

Die beschriebenen Nachteile des bekannten Herstellungsverfahrens sind insbesondere für die Herstellung von pharmazeutischen Produkten äußerst kritisch. In Bezug auf Hygiene (Verunreinigung durch Stärkestaub) und Reinheit (Kreuzkontamination) entspricht die Mogul-Technik nicht den Anforderungen der pharmazeutischen Standards (GMP-Richtlinien) und schränkt somit die Anwendungsgebiete von Gummibonbons bzw. Kautabletten als medizinische Darreichungsform stark ein.

Die Verwendung von festen, wiederverwendbaren Hohlformen (insbesondere aus Kunststoff) zum Gießen von derartigen Gelatineprodukten scheiterte bisher an wirtschaftlichen und technischen Gründen. Zwar sind solche Verfahren für die Herstellung von Zuckersüßwaren auf Basis von anderen, schnell gelierenden Hydrokolloiden (wie z.B. Pektin), die eine niedrige Viskosität der Gießmasse erlauben, bekannt. Die sensorischen Eigenschaften dieser Produkte unterscheiden sich aber deutlich, so dass sie aus Sicht des Verbrauchers keine Alternative zu Gelatineprodukten darstellen.

Eine Trocknung der bekannten Gießmassen auf Gelatinebasis ist in festen Hohlformen nicht möglich, da nur über die offene Oberseite Wasser entweichen könnte. Dies ist für eine vollständige und homogene Trocknung nicht ausreichend. Änderungen an der Rezeptur sind sehr schwierig, da verschiedene, zum Teil gegenläufige Rahmenbedingungen zu beachten sind: Einerseits müssen die rheologischen Eigenschaften der Gießmasse für den Gießvorgang geeignet sein, andererseits sollte das Endprodukt die typische Textur von Gelatineprodukten aufweisen, die vom Verbraucher erwartet wird.

Der vorliegenden Erfindung liegt daher die Aufgabe zugrunde, eine Gießmasse für die Herstellung von Gelatineprodukten bereitzustellen, die für das Gießen in feste Hohlformen geeignet ist.

Diese Aufgabe wird erfindungsgemäß gelöst durch eine Gießmasse der eingangs genannten Art, die eine homogene wässrige Lösung mit folgenden Bestandteilen umfasst:
- 4 bis 16 Gew.% Gelatine mit einem gelchromatographisch ermittelten mittleren Molekulargewicht von mindestens 130 kDa, bevorzugt mindestens 145 kDa, wobei der Anteil der Gelatine mit einem Molekulargewicht über 130 kDa mindestens 35 Gew.% beträgt, bevorzugt mindestens 45 Gew.%;
- 6 bis 76 Gew.% eines oder mehrerer Zuckeralkohole;
- 8 bis 40 Gew.% Glucosesirup mit einer Viskosität von weniger als 800 mPa·s, bevorzugt weniger als 700 mPa·s, gemessen bei einer Trockensubstanz von 80 Gew.% und einer Temperatur von 50 °C; und
- 0 bis 50 Gew.% Saccharose,
wobei der Glucosesirup und der oder die Zuckeralkohole zusammen 25 bis 76 Gew.% der wässrigen Lösung ausmachen, und wobei die wässrige Lösung eine Trockensubstanz von mindestens 78 Gew.% und/oder eine Wasseraktivität (a_{w}-Wert) von weniger als 0,75 aufweist.

Es hat sich überraschenderweise gezeigt, dass die erfindungsgemäße Gießmasse in der Lage ist, während des Abkühlens nach dem Gießen allein durch das Gelieren der Gelatine die für Gelatineprodukte typische Textur und Konsistenz zu erreichen, ohne dass hierfür in nennenswertem Umfang eine Trocknung, d.h. eine Abgabe von Wasser, erforderlich ist. Ermöglicht wird dies durch eine Gießmasse, die im Wesentlichen bereits denselben geringen Wassergehalt aufweist wie die herzustellenden Gelatineprodukte.

Trotz der im Vergleich zum Stand der Technik höheren Trockensubstanz weist die Gießmasse aufgrund ihrer Komponenten solche rheologischen Eigenschaften auf, die eine Verarbeitung in der üblichen Art und Weise erlauben. Entscheidend sind einerseits eine hinreichend niedrige Viskosität der heißen Gießmasse, und andererseits eine rasche Erstarrung des Systems beim Abkühlen. Nur so können, bei gleichzeitig guter Ausformbarkeit, unerwünschte Phänomene wie Fadenbildung und/oder Lufteinschlüsse vermieden werden.

Aufgrund dieser Eigenschaften kann die erfindungsgemäße Gießmasse bei der Herstellung von Gelatineprodukten in feste Hohlformen, insbesondere aus einem Kunststoffmaterial wie z.B. Silikon, gegossen werden. Neben den Vorteilen, die sich dabei aus dem Verzicht auf das bisher verwendete Stärkeformpuder ergeben, führt dies auch zu einer wesentlichen Verkürzung des Herstellungsverfahrens, da die erfindungsgemäße Gießmasse in der Regel bereits nach weniger als 60 Minuten fest genug ist, um ohne übermäßiges Anhaften entformt werden zu können. Demgegenüber benötigen die mit den bekannten Rezepturen in Stärkeformpuder gegossenen Produkte zwischen 24 und 72 Stunden für ihre Trocknung. Die erfindungsgemäße Gießmasse führt somit, bei derselben Stückzahl pro Zeiteinheit, zu einem deutlich reduzierten Platzbedarf für die abkühlenden Gummibonbons, was sowohl die Investitionen für die Hohlformen und den Lagerraum für die nicht mehr benötigte Trocknung, als auch die Betriebskosten (keine Stärketrocknung und Klimatisierung der Lagerräume) deutlich senkt.

Die erfindungsgemäße Gießmasse löst die gestellte Aufgabe durch das Zusammenspiel der enthaltenen Komponenten innerhalb der oben angegebenen Mengenbereiche. Ein wesentliches Merkmal der Erfindung ist dabei die Auswahl einer hochmolekularen Gelatine mit einem gelchromatographisch ermittelten mittleren Molekulargewicht von mindestens 130 kDa, wobei der Anteil der Gelatine mit einem Molekulargewicht über 130 kDa mindestens 35 Gew.% beträgt. Solche Gelatinen können aus verschiedenen kollagenhaltigen Materialien gewonnen werden, insbesondere aus Bindegewebe oder Knochen von Schweinen, Rindern, Geflügel oder Fischen.

Als weitere Komponenten können in der Gießmasse, insbesondere in der homogenen wässrigen Lösung, Aromastoffe, Farbstoffe und/oder Säuerungsmittel enthalten sein, wobei die typischen Mengenanteilen solcher Zusatzstoffe aus dem Stand der Technik bekannt sind. Als Säuerungsmittel werden die üblichen Genusssäuren, vorzugsweise Zitronensäure, eingesetzt.

Die erfindungsgemäße Gießmasse kann neben den vorstehend beschriebenen Komponenten auch noch weitere Bestandteile enthalten, so lange diese keinen negativen Einfluss auf die genannten vorteilhaften Eigenschaften der Gießmasse haben. Bei einer bevorzugten Ausführungsform umfasst die Gießmasse einen oder mehrere Nährstoffe und/oder pharmazeutische Wirkstoffe, wobei diese je nach Löslichkeit in der homogenen wässrigen Lösung gelöst oder darin dispergiert sein können. Im ersten Fall liegt der Anteil solcher Komponenten bevorzugt bei weniger als 45 Gew.%, weiter bevorzugt weniger als 35 Gew.%. Bevorzugte Nährstoffe in der erfindungsgemäßen Gießmasse sind ausgewählt aus Vitaminen, Mineralstoffen, Pflanzenextrakten und Peptiden, insbesondere Kollagenpeptiden (Kollagenhydrolysat). Bei den entsprechenden Gelatineprodukten kann es sich um angereicherte Süßwaren (Fortified Gummies) oder um Nahrungsergänzungsmittel handeln.

Die erfindungsgemäße Gießmasse kann auch zur Herstellung von Arzneimitteln eingesetzt werden (z.B. in Form von Kautabletten) und in diesem Fall pharmazeutisch Wirkstoffe enthalten, z.B. Schmerzmittel wie Acetylsalicylsäure, Paracetamol oder Ibuprofen. Die Eignung der Gießmasse zur Verwendung von festen Hohlformen ermöglicht im Gegensatz zur Mogul-Technik die Einhaltung der in diesem Bereich geltenden Hygienestandards (GMP-Richtlinien).

Die Gelatine kann in der wässrigen Lösung in einem Anteil von 4 bis 16 Gew.% enthalten sein, wobei ein Anteil von 5 bis 12 Gew.% bevorzugt ist, insbesondere von 6 bis 10 Gew.%. In diesem Bereich werden Gelatineprodukte mit einer typischen Textur, insbesondere einer hohen Elastizität, erhalten. Glucosesirup und Saccharose können in der wässrigen Lösung in einem Anteil von jeweils bis zu 40 Gew.% enthalten sein. Zur Herstellung von zuckerfreien Produkten können diese Komponenten jedoch auch entfallen und durch einen höheren Anteil an Zuckeralkoholen kompensiert werden.

Bei Verwendung von Glucosesirup weist dieser bevorzugt einer Viskosität von weniger als 800 mPa•s auf, weiter bevorzugt von weniger als 700 mPa•s, gemessen bei einer Trockensubstanz von 80 Gew.% und einer Temperatur von 50 °C. Es handelt sich günstigerweise um einen hoch hydrolysierten Glucosesirup mit einem Dextrose-Äquivalent von 50 oder mehr auf, bevorzugt von 60 oder mehr.

Außer im Fall von zuckerfreien Produkten enthält die wässrige Lösung in einem Anteil von 8 bis 40 Gew.% Glucosesirup, bevorzugt von 15 bis 28 Gew.%.

Saccharose, also handelsüblicher Zucker, ist bevorzugt in einem Anteil von 15 bis 45 Gew.% in der wässrigen Lösung enthalten (außer bei zuckerfreien Produkten), weiter bevorzugt von 20 bis 40 Gew.%.

Die wässrige Lösung umfasst ferner einen oder mehrere Zuckeralkohole in einem Anteil von 6 bis 76 Gew.%, bevorzugt von 10 bis 30 Gew.%. Durch die Verwendung dieser Zuckeraustauschstoffe kann einerseits die Menge an Zucker und/oder Glucosesirup reduziert werden, andererseits tragen die Zuckeralkohole auch zu den günstigen rheologischen Eigenschaften der Gießmasse bei.

Der oder die Zuckeralkohole sind vorzugsweise ausgewählt sind aus Sorbit, Mannit, Xylit, Erythrit und Glycerin, wobei Sorbit besonders bevorzugt ist. Gemäß einer Variante der Erfindung umfasst die homogene wässrige Lösung ferner ein oder mehrere weitere Hydrokolloide, insbesondere Pektin, um die Eigenschaften der Gelatineprodukte (z.B. Temperaturstabilität und Elastizität) zu modifizieren. Der Anteil weiterer Hydrokolloide ist vorzugsweise von 0,1 bis 10 Gew.%, insbesondere von 0,2 bis 5 Gew.%.

Die erfindungsgemäße Gießmasse eignet sich, wie oben beschrieben, zur Herstellung von Gelatineprodukten durch Gießen in feste Hohlformen, insbesondere aus einem Kunststoffmaterial.

Die vorliegende Erfindung betrifft daher auch ein Verfahren zur Herstellung von Gelatineprodukten, umfassend die Schritte:
- Herstellen einer erfindungsgemäßen Gießlösung;
- Gießen der Gießlösung bei einer Temperatur von 80 °C oder mehr in feste Hohlformen;
- Abkühlen der Gießlösung in den Hohlformen, um die Gelatineprodukte zu erhalten; und
- Entnehmen der Gelatineprodukte aus den Hohlformen.

Das Abkühlen der Gießlösung in den Hohlformen erfolgt bevorzugt innerhalb eines Zeitraums von weniger als 60 min, weiter bevorzugt weniger als 45 min. Dies stellt einen erheblichen Vorteil gegenüber dem Gießen in Hohlformen in einem Stärkeformpuder dar, wo die Gelatineprodukte üblicherweise erst nach einer Trocknungszeit von 24 bis 72 Stunden entnommen werden können.

Für die festen Hohlformen kann grundsätzlich jedes Material, insbesondere Kunststoffmaterial, eingesetzt werden, das temperaturstabil (bis ca. 95 °C) und für den Kontakt mit Lebensmitteln geeignet ist. Besonders bevorzugt sind Hohlformen aus Silikon, das aufgrund seiner Flexibilität eine leichte Entformung der Gelatineprodukte ermöglicht.

Weitere Beispiele für geeignete Kunststoffmaterialien sind z.B. Polycarbonat (PC) oder Polyethylenterephthalat (PET). Aus dünnen Folien dieser Kunststoffe können mittels Tiefziehen Hohlformen hergestellt werden, ähnlich wie die bekannten Blisterverpackungen für Medikamente. Diese Materialien sind kostengünstiger als Silikon, was die Möglichkeit eröffnet, individualisierte Hohlformen z.B. mit Beschriftungen herzustellen, welche in die Gelatineprodukte eingeprägt werden. Diese Hohlformen können dann nach relativ wenigen Produktionszyklen verworfen werden.

Nach dem Entnehmen der Gelatineprodukte aus den Hohlformen können diese je nach gewünschter Optik entweder mit einem Trennwachs behandelt werden oder mit Saccharose und/oder Zitronensäure bestäubt werden.

Ein Gegenstand der vorliegenden Erfindung sind auch die Gelatineprodukte, die nach dem erfindungsgemäßen Verfahren hergestellt sind. Trotz ihrer neuartigen Zusammensetzung weisen diese beim Verzehr die typische Textur auf, welche die Verbraucher bei Gelatineprodukten erwarten.

Wie bereits eingangs erwähnt, umfasst der Begriff Gelatineprodukte im Rahmen der vorliegenden Erfindung sämtliche Süßwaren, Nahrungsergänzungsmittel oder Arzneimittel mit der entsprechenden Zusammensetzung, unabhängig von ihrer äußeren Form. Typische Beispiele für solche Produkte sind Gummibonbons, Fruchtgummis, Fortified Gummies, Kautabletten usw.

Die erfindungsgemäßen Gelatineprodukte weisen bevorzugt eine Trockensubstanz von mindestens 78 Gew.% und/oder eine Wasseraktivität (a_{w}-Wert) von weniger als 0,75 auf. Wie bereits im Zusammenhang mit der erfindungsgemä-βen Gießmasse beschrieben, erfolgt während des Abkühlens und Gelierens keine oder nur eine unwesentliche Erhöhung der Trockensubstanz.

Diese und weitere Vorteile der Erfindung werden anhand der nachfolgenden Beispiele näher erläutert.

### Beispiele

Gemäß dem nachfolgend beschriebenen Verfahren wurden Gelatineprodukte hergestellt aus sechs verschiedenen erfindungsgemäßen Gießmassen (Beispiele 1 bis 6) und einer nicht erfindungsgemäßen Gießmasse als Vergleichsbeispiel.

Hierzu wurde zunächst die Gelatine in heißem Wasser (70 bis 80 °C) vollständig aufgelöst, der Sorbit und ggf. Glycerin zugegeben, homogen gemischt und nochmals auf 70 bis 80 °C erhitzt.

Parallel wurde ein so genannter Zuckerslurry hergestellt, indem Glucosesirup, Saccharose und Sorbit (sowie ggf. Pektin) in Wasser unter Druck auf mindestens 125 °C aufgekocht wurden. Der auf ca. 110 °C abgekühlte Zuckerslurry und die Gelatine-/Sorbitlösung wurden vereinigt und die Mischung unter Vakuum entgast und auf 80 °C abgekühlt. Beim Entgasen wurde auch der Wassergehalt der Zusammensetzung so weit reduziert, dass die Trockensubstanz bei mindestens 78 Gew.% lag.

Dem Ansatz wurde dann Zitronensäure als Säuerungsmittel hinzugefügt und die Gießmasse in Hohlformen aus Silikon (Beispiele 1 bis 5 und Vergleichsbeispiel) bzw. in Hohlformen in einem PET-Blister (Beispiel 6) abgefüllt. Hierfür wurde eine Laborgießanlage der Firma Winkler und Dünnebier Süßwarenmaschinen GmbH verwendet. Die Füllmenge lag jeweils zwischen 1 und 5 g.

Nach einer Abkühlzeit von maximal 60 min bei unter 12 °C Umgebungstemperatur konnten die erfindungsgemäßen Gelatineprodukte (Gummibonbons/Kautabletten) entformt werden. Diese können anschließend nach Belieben mit einem Trennwachs geölt oder mit Zucker und Zitronensäure bestäubt und dann verpackt werden.

In der nachfolgenden Tabelle 1 sind für die Beispiele 1 bis 6 und das Vergleichsbeispiel die jeweiligen Zusammensetzungen der fertigen Gießmasse vor dem Abfüllen in die Hohlformen angegeben. Ferner sind verschiedene Parameter der Gießmasse, des Herstellungsverfahrens und der hergestellten Gelatineprodukte angegeben.

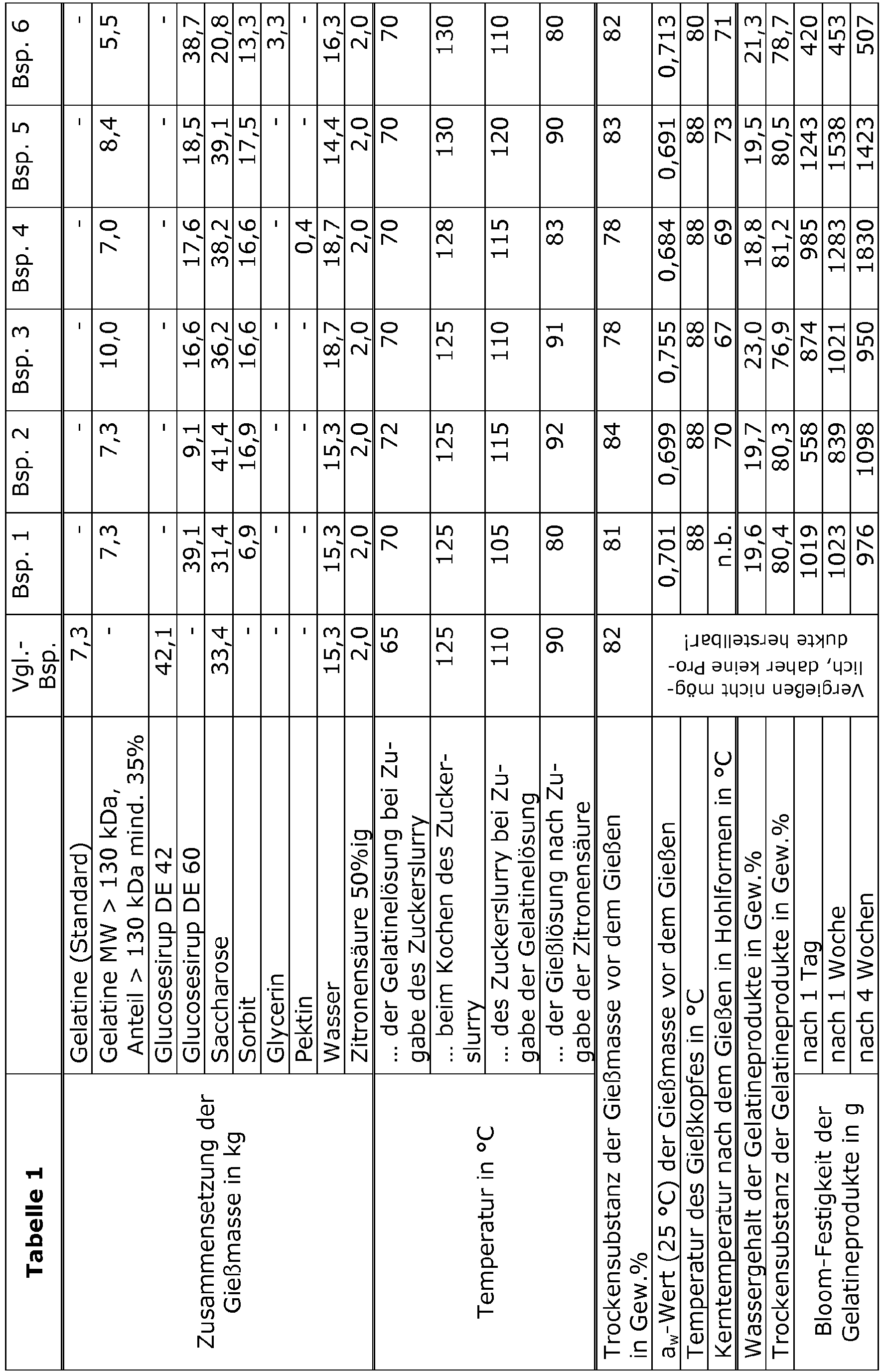

Das Vergleichsbeispiel entspricht der typischen Rezeptur für eine Mogul-Anlage gemäß dem Stand der Technik, mit einer Standardgelatine, niedrig hydrolysiertem Glucosesirup (DE 42), und ohne Zuckeralkohole. Diese Gießmasse ließ sich aufgrund von Fadenbildung und Lufteinschlüssen nur schwer vergießen und war nach einer Abkühlzeit von 60 min immer noch zu weich und zu klebrig, um aus den Silikonformen entnommen werden zu können. Bei den Gelatineprodukten gemäß Beispiel 1, die aus einer Gießmasse mit hochmolekularer Gelatine, hoch hydrolysiertem Glucosesirup (DE 60) und Sorbit hergestellt wurden, war dies bereits nach weniger als 60 min möglich.

Bei den Beispielen 2 bis 5, welche modifizierte Anteile an Saccharose, Glucosesirup und Sorbit enthielten, sowie bei Beispiel 6 mit zusätzlichem Glycerin, war eine Entformung der Produkte bereits nach maximal 40 min möglich, wobei die Endprodukte zum Teil unterschiedliche Texturen aufwiesen (z.B. kürzere, festere Textur bei Beispiel 4, welches zusätzlich Pektin enthielt).

Durch Variationen der Rezeptur der erfindungsgemäßen Gießmasse konnten die Textureigenschaften der hergestellten Gelatineprodukte modifiziert werden, in Abhängigkeit von Trockensubstanzgehalt, Gelatinedosierung und Zuckerzusammensetzung.

## Patentansprüche

1. Gießmasse für die Herstellung von Gelatineprodukten durch Gießen in feste Hohlformen, umfassend eine homogene wässrige Lösung mit folgenden Bestandteilen:
- 4 bis 16 Gew.% Gelatine mit einem gelchromatographisch ermittelten mittleren Molekulargewicht von mindestens 130 kDa, bevorzugt mindestens 145 kDa, wobei der Anteil der Gelatine mit einem Molekulargewicht über 130 kDa mindestens 35 Gew.% beträgt, bevorzugt mindestens 45 Gew.%;
- 6 bis 76 Gew.% eines oder mehrerer Zuckeralkohole;
- 8 bis 40 Gew.% Glucosesirup mit einer Viskosität von weniger als 800 mPa·s, bevorzugt weniger als 700 mPa·s, gemessen bei einer Trockensubstanz von 80 Gew.% und einer Temperatur von 50 °C; und
- 0 bis 50 Gew.% Saccharose,
wobei der Glucosesirup und der oder die Zuckeralkohole zusammen 25 bis 76 Gew.% der wässrigen Lösung ausmachen, und wobei die wässrige Lösung eine Trockensubstanz von mindestens 78 Gew.% und/oder eine Wasseraktivität (a_{w}-Wert) von weniger als 0,75 aufweist.

2. Gießmasse nach Anspruch 1, wobei die homogene wässrige Lösung ferner einen oder mehrere Aromastoffe, Farbstoffe und/oder Säuerungsmittel enthält.

3. Gießmasse nach Anspruch 1 oder 2, wobei die homogene wässrige Lösung ferner einen oder mehrere Nährstoffe und/oder pharmazeutische Wirkstoffe enthält, bevorzugt in einem Anteil von weniger als 45 Gew.%, insbesondere weniger als 35 Gew.%.

4. Gießmasse nach einem der vorhergehenden Ansprüche, ferner umfassend einen oder mehrere unlösliche Bestandteile, die in der wässrigen Lösung dispergiert sind, und die bevorzugt ausgewählt sind aus Nährstoffen und pharmazeutischen Wirkstoffen.

5. Gießmasse nach Anspruch 3 oder 4, wobei die Nährstoffe ausgewählt sind aus Vitaminen, Mineralstoffen, Pflanzenextrakten und Peptiden, insbesondere Kollagenpeptiden.

6. Gießmasse nach einem der vorhergehenden Ansprüche, wobei die Gelatine in der wässrigen Lösung in einem Anteil von 5 bis 12 Gew.%, bevorzugt von 6 bis 10 Gew.%, enthalten ist.

7. Gießmasse nach einem der vorhergehenden Ansprüche, wobei der Glucosesirup ein Dextrose-Äquivalent von 50 oder mehr, bevorzugt von 60 oder mehr, aufweist.

8. Gießmasse nach einem der vorhergehenden Ansprüche, wobei der Glucosesirup in der wässrigen Lösung in einem Anteil von 15 bis 28 Gew.% enthalten ist; und/oder wobei die Saccharose in der wässrigen Lösung in einem Anteil von 15 bis 45 Gew.%, bevorzugt von 20 bis 40 Gew.%, enthalten ist.

9. Gießmasse nach einem der vorhergehenden Ansprüche, wobei der oder die Zuckeralkohole in der wässrigen Lösung in einem Anteil von 10 bis 30 Gew.% enthalten sind; und/oder wobei der oder die Zuckeralkohole ausgewählt sind aus Sorbit, Mannit, Erythrit und Glycerin, bevorzugt Sorbit.

10. Gießmasse nach einem der vorhergehenden Ansprüche, wobei die homogene wässrige Lösung ferner ein oder mehrere weitere Hydrokolloide, insbesondere Pektin, umfasst, bevorzugt in einem Anteil von 0,1 bis 10 Gew.%, insbesondere von 0,2 bis 5 Gew.%.

11. Verfahren zur Herstellung von Gelatineprodukten, umfassend die Schritte:
- Herstellen einer Gießlösung nach einem der Ansprüche 1 bis 10;
- Gießen der Gießlösung bei einer Temperatur von 80 °C oder mehr in feste Hohlformen;
- Abkühlen der Gießlösung in den Hohlformen, um die Gelatineprodukte zu erhalten; und
- Entnehmen der Gelatineprodukte aus den Hohlformen.

12. Verfahren nach Anspruch 11, wobei das Abkühlen der Gießlösung in den Hohlformen innerhalb eines Zeitraums von weniger als 60 min, bevorzugt weniger als 45 min, erfolgt.

13. Verfahren nach Anspruch 11 oder 12, wobei die festen Hohlformen aus einem Kunststoffmaterial bestehen, das bevorzugt ausgewählt ist aus Silikon, Polycarbonat oder Polyethylenterephthalat.

14. Verfahren nach einem der Ansprüche 11 bis 13, wobei die Gelatineprodukte nach dem Entnehmen aus den Hohlformen mit einem Trennwachs behandelt werden, oder mit Saccharose und/oder Zitronensäure bestäubt werden.

15. Gelatineprodukte, hergestellt gemäß dem Verfahren nach einem der Ansprüche 11 bis 14.

## Claims

1. Casting compound for producing gelatin products by pouring into solid hollow moulds, comprising a homogeneous aqueous solution containing the following constituents:
- 4 to 16 wt % of gelatin having a mean molecular weight, determined by gel chromatography, of at least 130 kDa, preferably at least 145 kDa, wherein the proportion of the gelatin having a molecular weight of more than 130 kDa is at least 35 wt %, preferably at least 45 wt %;
- 6 to 76 wt % of one or more sugar alcohols;
- 8 to 40 wt % of glucose syrup with a viscosity of less than 800 mPa·s, preferably less than 700 mPa·s, measured with a dry matter content of 80 wt % and at a temperature of 50°C; and
- 0 to 50 wt % of sucrose,
wherein the glucose syrup and the sugar alcohol(s) together make up 25 to 76 wt % of the aqueous solution, and wherein the aqueous solution has a dry matter content of at least 78 wt % and/or a water activity (a_{w} value) of less than 0.75.

2. Casting compound according to claim 1, wherein the homogeneous aqueous solution also contains one or more flavourings, colourings and/or acidifiers.

3. Casting compound according to claim 1 or 2, wherein the homogeneous aqueous solution also contains one or more nutrients and/or pharmaceutical active substances, preferably in a proportion of less than 45 wt %, in particular less than 35 wt %.

4. Casting compound according to any one of the preceding claims, also comprising one or more insoluble constituents, which are dispersed in the aqueous solution and which are preferably selected from nutrients and pharmaceutical active substances.

5. Casting compound according to claim 3 or 4, wherein the nutrients are selected from vitamins, minerals, plant extracts and peptides, in particular collagen peptides.

6. Casting compound according to any one of the preceding claims,
wherein the gelatin is contained in the aqueous solution in a proportion of from 5 to 12 wt %, preferably from 6 to 10 wt %.

7. Casting compound according to any one of the preceding claims,
wherein the glucose syrup has a dextrose equivalent of 50 or more, preferably of 60 or more.

8. Casting compound according to any one of the preceding claims,
wherein the glucose syrup is contained in the aqueous solution in a proportion of from 15 to 28 wt %; and/or wherein the sucrose is contained in the aqueous solution in a proportion of from 15 to 45 wt %, preferably from 20 to 40 wt %.

9. Casting compound according to any one of the preceding claims,
wherein the sugar alcohol(s) is/are contained in the aqueous solution in a proportion of from 10 to 30 wt %; and/or wherein the sugar alcohol(s) is/are selected from sorbitol, mannitol, erythritol and glycerol, preferably sorbitol.

10. Casting compound according to any one of the preceding claims,
wherein the homogeneous aqueous solution also comprises one or more further hydrocolloids, in particular pectin, preferably in a proportion of from 0.1 to 10 wt %, in particular from 0.2 to to 5 wt %.

11. Method for producing gelatin products, said method comprising the following steps:
- producing a casting solution according to any one of claims 1 to 10;
- pouring the casting solution at a temperature of 80°C or more into solid hollow moulds;
- cooling the casting solution in the hollow moulds in order to obtain the gelatin products; and
- removing the gelatin products from the hollow moulds.

12. Method according to claim 11, wherein the casting solution cools in the hollow moulds within a period of time of less than 60 min., more preferably less than 45 min.

13. Method according to claim 11 or 12, wherein the solid hollow moulds consist of a plastics material which is preferably selected from silicone, polycarbonate or polyethylene terephthalate.

14. Method according to any one of claims 11 to 13, wherein the gelatin products are treated with a release wax or are dusted with sucrose and/or citric acid after having been removed from the hollow moulds.

15. Gelatin products, produced in accordance with the method according to any one of claims 11 to 14.

## Revendications

1. Masse de coulée pour la fabrication de produits gélatineux par coulée dans des moules creux rigides, comprenant une solution aqueuse homogène ayant les composants suivants :
- 4 à 16 % en poids de gélatine ayant un poids moléculaire moyen déterminé par chromatographie sur gel d'au moins 130 kDa, de préférence d'au moins 145 kDa, la proportion de gélatine ayant un poids moléculaire supérieur à 130 kDa étant d'au moins 35 % en poids, de préférence d'au moins 45 % en poids ;
- 6 à 76 % en poids d'un ou de plusieurs alcools de sucre ;
- 8 à 40 % en poids de sirop de glucose ayant une viscosité inférieure à 800 mPa·s, de préférence inférieure à 700 mPa·s, mesurée pour une matière sèche de 80 % en poids et à une température de 50 °C ; et
- 0 à 50 % en poids de saccharose,
le sirop de glucose et le ou les alcools de sucre constituant ensemble 25 à 76 % en poids de la solution aqueuse, et la solution aqueuse présentant une matière sèche d'au moins 78 % en poids et/ou une activité aqueuse (valeur aw) inférieure à 0,75.

2. Masse de coulée selon la revendication 1, dans laquelle la solution aqueuse homogène comprend en outre un ou plusieurs arômes, colorants et/ou agents acidifiants.

3. Masse de coulée selon la revendication 1 ou 2, dans laquelle la solution aqueuse homogène comprend en outre un ou plusieurs nutriments et/ou principes actifs pharmaceutiques, de préférence en une proportion inférieure à 45 % en poids, en particulier inférieure à 35 % en poids.

4. Masse de coulée selon l'une des revendications précédentes, comprenant en outre un ou plusieurs composants insolubles qui sont dispersés dans la solution aqueuse et qui sont de préférence choisis parmi les nutriments et les principes actifs pharmaceutiques.

5. Masse de coulée selon la revendication 3 ou 4, dans laquelle les nutriments sont choisis parmi les vitamines, les minéraux, les extraits végétaux et les peptides, en particulier les peptides de collagène.

6. Masse de coulée selon l'une des revendications précédentes, dans laquelle la gélatine est présente dans la solution aqueuse en une proportion de 5 à 12 % en poids, de préférence de 6 à 10 % en poids.

7. Masse de coulée selon l'une des revendications précédentes, dans laquelle le sirop de glucose présente un équivalent de dextrose de 50 ou plus, de préférence de 60 ou plus.

8. Masse de coulée selon l'une des revendications précédentes, dans laquelle le sirop de glucose est présent dans la solution aqueuse en une proportion de 15 à 28 % en poids ; et/ou le saccharose est présent dans la solution aqueuse en une proportion de 15 à 45 % en poids, de préférence de 20 à 40 % en poids.

9. Masse de coulée selon l'une des revendications précédentes, dans laquelle le ou les alcools de sucre sont présents dans la solution aqueuse en une proportion de 10 à 30 % en poids ; et/ou le ou les alcools de sucre sont choisis parmi le sorbitol, le mannitol, l'érythritol et le glycérol, de préférence le sorbitol.

10. Masse de coulée selon l'une des revendications précédentes, dans laquelle la solution aqueuse homogène comprend en outre un ou plusieurs autres hydrocolloïdes, en particulier de la pectine, de préférence en une proportion de 0,1 à 10 % en poids, en particulier de 0,2 à 5 % en poids.

11. Procédé de fabrication de produits gélatineux, comprenant les étapes de :
- fabrication d'une solution de coulée selon l'une des revendications 1 à 10 ;
- coulée de la solution de coulée à une température de 80 °C ou plus dans des moules creux rigides ;
- refroidissement de la solution de coulée dans des moules creux pour obtenir les produits gélatineux ; et
- retrait des produits gélatineux des moules creux.

12. Procédé selon la revendication 11, dans lequel le refroidissement de la solution de coulée dans les moules creux s'effectue dans un laps de temps inférieur à 60 min, de préférence inférieur à 45 min.

13. Procédé selon la revendication 11 ou 12, dans lequel les moules creux rigides se composent d'un matériau plastique, qui est choisi de préférence parmi la silicone, le polycarbonate ou le polyéthylène téréphtalate.

14. Procédé selon l'une des revendications 11 à 13, dans lequel les produits gélatineux sont traités après retrait des moules creux avec une cire de séparation, ou sont saupoudrés de saccharose et/ou d'acide citrique.

15. Produits gélatineux fabriqués d'après le procédé selon l'une des revendications 11 à 14.
